# EUROPEAN PATENT SPECIFICATION

(11) **EP 0 611 820 B1**
(45) Date of publication and mention of the grant of the patent: **20.10.1999**
(21) Application number: 94200334.4
(22) Date of filing: 15.02.1994
(51) Int. Cl.: C12M 1/26, C12M 1/12

(54) **Method of inoculating a liquid and inoculation vessel and device for the application of the method**
Verfahren zur Inokulation eine Flüssigkeit, und Inokulationsgefäss und Vorrichtung für die Anwendung dieses Verfahrens
Procédé d'inoculation d'un liquide, et un récipient d'inoculation et dispositif pour application de ce procédé

(30) Priority: 17.02.1993 NL 9300302
(43) Date of publication of application: 24.08.1994
(73) Proprietor: TUCHENHAGEN NEDERLAND (TNL) B.V., 7213 AL Gorssel (NL)
(72) Inventor: De Jong, Lambertus, NL-7261 WL Ruurlo (NL)
(74) Representative: Bakker, Gilles Egbert, Ir.

(56) References cited:
- EP-A- 0 281 547
- DE-C- 3 935 050
- FR-A- 361 317

## Description

The invention relates to a method of inoculating a liquid, at which the inoculant placed in one or more closed containers is brought into a inoculation vessel, the containers are opened inside the inoculation vessel and the inoculant from the inoculation vessel is transferred to a liquid in a reaction chamber, and to an inoculation vessel and a device for the application of the method.

When inoculating a liquid such as milk to prepare yoghurt or cheese starter for cheese making, or any other bacterial of fermentation processes, a high degree of hygiene is generally demanded. Such requirement is not only necessary because the created product is often a food product or a food ingredient, or else meant to come into contact with human beings, so that decay or contamination cannot be allowed, but also because serious damage may occur when the liquid does not undergo the desired reaction or fermentation. In the latter case the liquid tends to be worthless, as a result of which, besides the loss of the liquid, additional costs arise in order to discard the liquid, which may lead, for instance, to a very substantial pollution tax. Moreover, a thorough cleaning of the installation concerned, and possibly of the installations close to it as well, will be required, which not only does entail expenses but also loss of production.

Finally, it is even possible, for instance in this case of contamination by bacteria or phages, that a new inoculation stock has to be introduced.

Although, as a result of this, much attention is paid to hygiene when inoculating liquids, it has been an unsolved problem how to prevent air-borne contaminations.

For instance, in practice a number of containers in an inoculation funnel or vessel are disinfected by means of a disinfectant, whereupon such disinfectant is discarded and the inoculant is carried from the containers towards the reaction vessel. During this process, however, there is the possibility that a contamination coming from the atmosphere may have access to the inoculant.

So the French Patent Specification FR-A-361 317 shows an apparatus in which an ampulla and a weight for breaking the ampulla are both sterilized by a sterilizing liquid and rinsed with pure water, after which they fall down in a guide, the weight above the ampulla, so that the latter breaks spreading its content, which contains the inoculant. This is a rather complicated and time consuming process and has the disadvantage of bringing the ampulla's splinters into the apparatus.

The German Patent Specification DE-C-39 35 050 describes a device for sterile conveying vegetable based cell cultures to a reaction vessel by means of a threefold conductor system which may be interconnected. Such a system is far from simple and has to be kept sterile itself, which may induce contamination risks and consumption of labour and time.

European Patent Application EP-A-0 281 547 depicts a device for breeding micro-organisms in which air is used, which is filtered when entering the breeder and when leaving it.

All three of these publications do not show simple and effective means to guarantee the inoculation and its immediate environment is perfectly sterile when being added to the liquid.

A further disadvantage of the often applied method was that the disinfectant simply was poured, for instance from a pail, on the containers located in an open funnel, which frequently gave rise to spilling.

The invention provides a method, which effectively excludes the possibility of air-borne contamination of the inoculative vessel and prevents spilling.

Therefore, it is provided, in accordance with the invention, that the inoculative vessel comes as a sealable inoculation vessel which, after the containers have been placed inside, is sealed and, after disinfection, is filled with sterile gas, whereupon the inoculant is conveyed into the reaction chamber.

In order to prevent contamination from the air above the liquid in the reaction chamber as well, in applying the invention it is preferably provided that the sterile gas is added under pressure, to both the reaction chamber and the inoculation vessel.

When using a sealed inoculation vessel it must be ensured that the air in said inoculation vessel is completely dispelled after the containers carrying the inoculant have been placed inside.

A second disadvantage can be that the disinfectant, even after the inoculation vessel has been emptied, may have been left in ducts and thus may have access to the liquid to be inoculated.

Said disadvantages are removed in accordance with a further elaboration of the invention by providing that the disinfection of the inoculation vessel takes place from underneath via a pipe that connects the inoculation vessel with the reaction chamber, while feeding and drainage of a disinfectant take place immediately above a stop-valve located in said pipe.

The invention also comprises an inoculation vessel for the application of the method. A known inoculation vessel is provided with one or more holding devices for the container of an inoculant and an opening device that is movable relative to the holding devices. In order to make such an inoculation vessel suitable for applying the invention it is, in accordance with the invention, provided that the inoculation vessel has one or more air-tight sealable openings for bringing in full and discarding empty containers and a mechanism for moving the holding devices and the opening devices relatively to each other, through an air-tight sealable wall.

In order to enable a full flow-through of cleaning liquid or disinfectant, it is, in accordance with a further elaboration of the invention, provided that the inoculation vessel has a top connection and a bottom connection.

Furthermore, the invention comprises a device for applying the method, which device has been provided with an inoculation vessel as specified hereinabove.

In order to achieve the aforementioned purposes of the invention, such a device is characterised by a source of sterile gas under a constant overpressure, which, by means of a duct and valve system, is connectable to the inoculation vessel and the reaction chamber, which source is provided with a gauge in order to open the air exhaust valve in case of a pressure higher than said constant overpressure.

A simple and effective embodiment of the source of sterile gas is, in accordance with a further elaboration of the invention, constituted in that a sterile filter, that as a source of sterile gas is located in a duct of supplied air, is connected with the duct and valve system and that the air exhaust valve is located on that side of the filter that is not facing the duct and valve system. By this means it is also achieved that, when the sterile gas flows back as occurs when filling the reaction chamber with liquid, the filter is blown clean in a counter current direction, which considerably prolongs the filter's life. Naturally such a filter is generally preceded by an oil or liquid filter and a coarser leading filter, while it is also possible to provide that the filter can be sterilized by means of steam.

In the food industry and actually the entire fermentation industry it is customary that the entire plant may be disinfected or sterilised by means of a disinfectant. In applying the invention, care should be taken that the disinfectant is discharged as completely as possible and that access to the reaction chamber is minimized. Although, in some cases, for instance during the fermentation of milk into yoghurt, it is possible to use a disinfectant that is binded and rendered harmless by the product, it remains advisable to remove the disinfectant as much as possible. In applying the invention, this is preferable achieved by providing a feeder for a disinfectant, which , through one or more valves, is connected to the under side of the inoculation vessel, and a drain for a disinfectant which, by means of valves, is connectable to the under side of the inoculation vessel.

It is of course also possible to apply the invention in a more simple form, simply by feeding under pressure a disinfectant to the under side of the inoculation vessel containing inoculant containers and thus disinfecting it, whereupon the disinfectant can be drained and, if need be, sterile gas can be admitted under pressure.

A device in accordance with the invention should in the customary way be suitable for being rinsed clean by means of a cleansing agent. It is in particular to be recommended to use the so-called 'cleaning in place', because in doing so a much more reliable cleaning is possible than when parts are disconnected and cleaned individually. If, however, such cleaning should be desired, it is recommended, when applying the invention to ensure that the feeding of the sterile gas under pressure and that of the cleansing agent do not interfere with each other.

Therefore, in accordance with a further characterization of the invention, it is preferably provided that there is a device, reacting on a liquid level, which closes the feeder of sterile gas when the cleansing agent reaches a predetermined level on said device reacting on a liquid level, and also that there are additional control valves that direct the cleansing agent through the inoculation vessel and the reaction chamber and from there ensure its removal.

The invention is further elucidated in the following by means of a drawing in which:
fig.1 shows a view of an inoculation vessel in accordance with the invention;
fig.2 shows a vertical view thereof, where a part of the inside mechanism is to be seen; and
fig. 3 shows a flow diagram of a device in accordance with the invention.

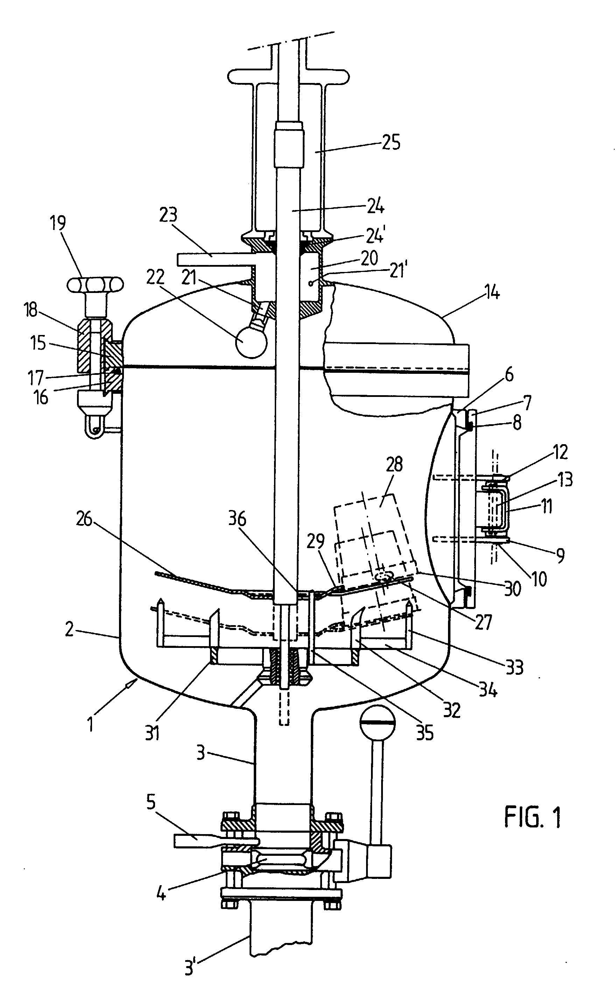

In fig. 1 by 1 is indicated an inoculation vessel in general, that has a bottom part 2, with a bottom drain 3, on the under side of which is a stop-valve 4 and right above a connection 5. The inoculation vessel 1 has an opening 6, on which a cap 7 fits, which, by means of an O-ring 8 can make a sealing. The cap 7 is by means of brackets 9 and shaft 10 connected to an arm 11, which itself carries another shaft 12 with a fixing bracket 13 for the cap 7. This cap can, in a known manner, be held down air-tight by means of a clamp.

On the bottom part 2 is an top part 14, where the flanges 15 and 16 together with an O-ring 17 form a sealing, which are fastened together by means of clamps 18 with rotating grips 19. On the upper side of the top part 14 there is a small space 20 which, via a duct 23, forms the upper outlet by means of one or more openings 21 which, optionally by means of a spray head 22 provided with small holes, not drawn, are in connection with the inner chamber of the inoculation vessel 1.

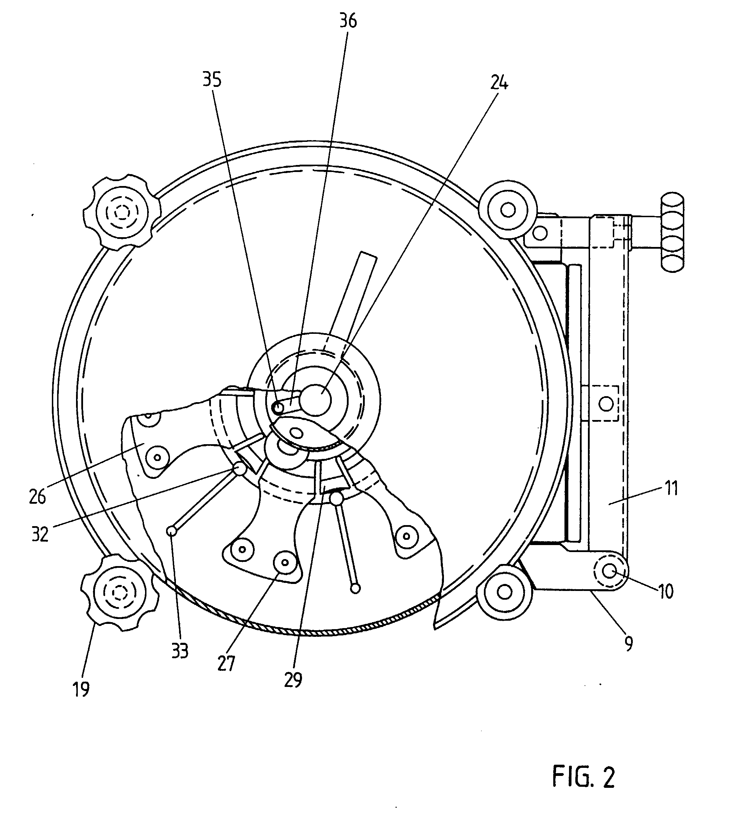

Through the inoculation vessel 1 runs a central bar 24, above which there is an electro-pneumatic device 25, only indicated in the drawing, that can move the bar 24 back and forth through a packing 24', as is indicated by dotted lines at the lower end. Onto the bar a plateau 26 is fixedly mounted, which, as is more in particular shown in fig. 2, is provided with retaining lips 29 and small holding rollers 27 in order to hold small cup-shaped containers 28. These small containers contain sterilized inoculant and are provided with rims 30 which ensure a grip.

To the inoculation vessel 1 a carrying ring 31 is fixedly connected, which has piercing or opening pins 32 and, through supporting devices 34, ventilation pins 33. When the bar 24 is moved downwards, the pins 32 and 33 will perforate the bottom of the small containers 28 and make openings in it, while the pins 32 have a thus shaped sharp upper end that an oval-shaped hole is created, and the small pins 33, which serve for ventilation, have a small diameter, so that they make a smaller hole. A guiding pin 35, which sticks into a oblong opening 36 in the platform 26, ensures that the opening and ventilation pins 32 and 33 are in alignment with the containers 28.

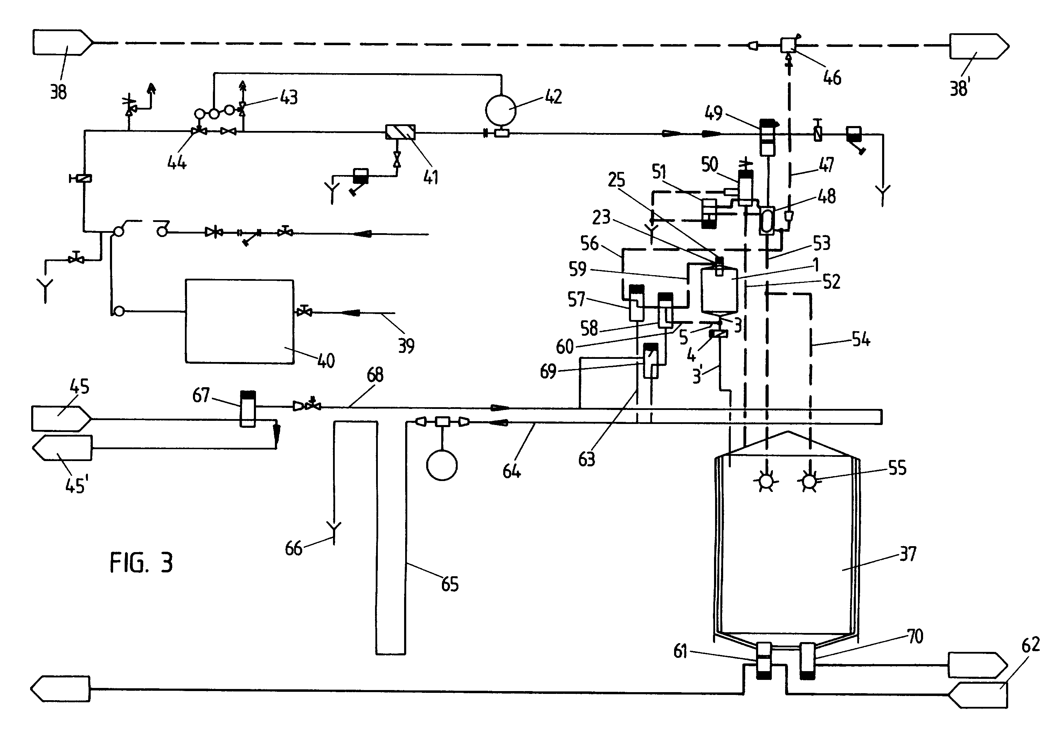

When the containers 28 are perforated, the inoculant flows through the drain 3 via the then opened sealing 4, through the duct 3' to a reaction vessel 37, which has been drawn in fig. 3 as have the ducts and valves involved.

The represented inoculation vessel has the property that, when it is filled from the bottom part with liquid, said liquid can be removed via the space 20, for the purpose of which on the very top of the vessel one or more additional openings 21 are situated. Due to this set up it is possible to dispel all gas from the inoculation vessel when cleaning or disinfecting.

After the usual cleaning with cleansing agent, for instance 1.5% caustic soda of 85° C with a low concentration of disinfectant, an overpressure of sterile gas will be created in the vessel until the inoculation vessel has to be filled. When subsequently the cap 7 is opened, this overpressure (e.g. 0.3 bar) is immediately relieved and an interruption of the connection with the feeding of sterile gas ensures that the inoculation vessel remains under atmospheric pressure. After the containers 28 have been placed inside and cap 7 has been sealed again, the disinfection of the inoculation vessel 1 takes place, when the containers 28 are located inside, because these containers can also be contaminated on the outside. As a sterilizing agent e.g. a solution of 0.35% peracetic acid is used. Subsequently sterile gas is again added under pressure, whereupon the perforation of the containers 28 can be effected and inoculant can be drained via duct 3, valve 4 and duct 3'. After the connection with the reaction chamber has been closed again, the inoculation vessel can be cleaned with rinsing liquid, e.g. comprising of hot diluted lye (85° C), naturally after the containers have been removed.

In the diagram of fig. 3 all valves and stop-valves can be electronically operated, for instance electropneumatically. As this diagram shows, a device in accordance with the invention is in the first place provided with a feeder 38 of a cleaning liquid which via a valve 46 can be fed to the system and which runs via an outlet 38' to other plants to be cleaned.

Furthermore the device is provided with a feeder 39 of air under pressure which runs via a liquid filter, carbon filter and, if need be, a coarse leading filter 40 to a filter 41, which provides a connection with the system through a valve 49. In the duct beyond the filter 41 a pressure gauge 42 is fitted, which, in case of a pressure higher than the predetermined pressure, opens an air exhaust valve 43 and closes the feeder 44. This, for instance, is necessary when the reaction chamber 37 is filled with a liquid and the sterile gas (in this case sterile air) is dispelled.

Furthermore, there is a feeder 45 for a disinfectant, which runs through to a drainage 45' for other plants.

When, via the valve 46 and the duct 47, cleansing liquid is fed to a float device 48, the latter will stop the flow of sterile gas under pressure by means of valve 49. Furthermore, via the valves 50 and 51, the cleaning liquid is admitted by a channel that admits passage of only a small quantity of liquid and that runs to the drain related to it, so that said valves are cleaned. The liquid acting on the float device 48 goes, via the duct 56 to the valve 57 and from there, via the valve 58 and ducts 59,60, to the top and under side of the inoculation vessel 1, and from there also via duct 3 and stop-valve 4 and duct 3' to the reaction chamber 37. In addition, via the duct 53 and its subsidiary duct 54, liquid is transferred to the sprayers 55, which causes the entire inner wall of the reaction chamber to be cleaned. Via the valve 61 the liquid is conveyed from the reaction chamber to the outlet 62.

After the plant has thus been cleaned, the cleaning liquid is drained via ducts 59 and 60, valve 58, reversed valve 57, valve 69, ducts 63 and 64, a descending duct loop 65 in order to prevent gas blow through, and finally via drain 66. Subsequently the containers 28 are brought into the inoculation vessel 1 after cap 7 has been opened, whereupon the valve 4 is closed and the stop-valve 57 remains reversed so that the connection with air under pressure, which was capable of being readmitted after the float device 48 had lost its liquid, is cut off.

Then the cups are placed in the inoculation vessel 1 and said vessel is disinfected via feeder 45, valve 67, duct 68, valves 69,58, ducts 5,3 to the vessel 1, from which the drainage flows via duct 23, duct 59, valve 58,57, and ducts 63,64,65 to drain 66.

Drainage in order to remove the disinfectant subsequently takes place via duct 5, valves 58,69 and ducts 64,65 to drain 66.

The inoculant can now be released because the electro-pneumatic valve 25 is operated and the pins 32 and 33 perforate the containers 28, while the valve 4 is opened and the inoculant, subject to gravitational force, can flow to the reaction chamber, because by now valve 57 admits passage to valve 58 and as a result the overpressure is introduced to the top of the inoculation vessel 1, so that said vessel is under the same pressure as the reaction chamber 37.

The reaction chamber has now been inoculated and after the completion of the reaction or fermentation, for instance the making of cottage cheese, yoghurt, curd or buttermilk, the drainage takes place via stop-valve 70, which is also connected to a bottling machine and to a device for maintaining the overpressure prevailing in the entire plant of, applicable to this example, 0.3 bar.

In the following table the valve positions are indicated.

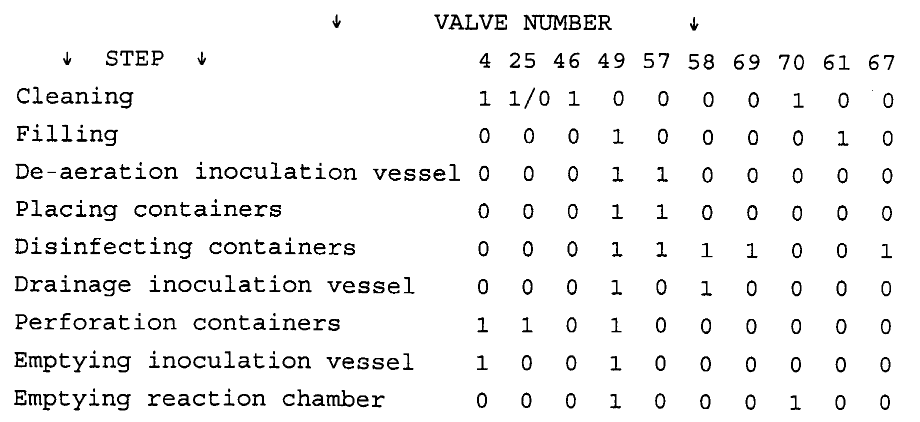

It will be clear that, when applying the invention, such a complicated system as is represented in fig.3 is not always necessary and that a partly manual operation is possible as well.

The system as shown here, however, has the important advantage that, after the containers have been brought into the inoculation vessel, everything can be controlled from a central controlling device, which generally allows for a saving of work and time. It is, however, also possible to carry out the disinfection by, for instance, connecting a portable pressure tank to the connection 5 and after the completion of the disinfection by effecting the drainage of the disinfectant via said connection.

Furthermore, in applying the invention, as is customary when rinsing for a first rinse, rinsing water of a previous rinse is used and for the final rinse a clean rinsing water, to which a small quantity of disinfectant, for instance 0.02% peracetic acid, has been added.

The invention is amply applicable in those cases where contamination and pollution have to be prevented to a high degree of certainty. In addition application is also possible when, for instance, producing antibiotics by means of bacteria, while the high degree of hygiene also allows to omit the use of preservatives, which may be a medical advantage as well.

## Claims

1. Method of inoculating a liquid, at which the inoculant placed in one or more closed containers (28) is brought into an inoculation vessel (1), the containers are opened inside the inoculation vessel and the inoculant from the inoculation vessel is transferred to the liquid in a reaction chamber (37), **characterized in that** the inoculation vessel comes as a sealable inoculation vessel (1), which, after the containers have been placed inside, is sealed and, after disinfection, is filled with sterile gas, whereupon the inoculant is conveyed into the reaction chamber.

2. Method as claimed in claim 1, **characterized in that** the sterile gas is added under pressure to both the reaction chamber (37) and the inoculation vessel (1).

3. Method as claimed in claim 1 or 2, relating to the disinfection of the inoculation vessel, **characterized in that** the disinfection of the inoculation vessel takes place from underneath via a pipe (3) that connects the inoculation vessel with the reaction chamber, while feeding and drainage (5) of a disinfectant take place immediately above a stop-valve (4) located in said pipe.

4. Inoculation vessel for the application of the method as claimed in one or more of the claims 1-3, provided with one or more holding devices (27,29) for the containers (28) and one or more opening devices that are movable relative to the holding devices, **characterized in that** the inoculation vessel (1) has one or more air-tight sealable openings (6) for bringing in full and discarding empty containers (28) and a mechanism (25) for moving the holding devices and the opening devices relatively to each other, through an air-tight sealable wall (24').

5. Inoculation vessel as claimed in claim 4, **characterized in that** it has a top connection (23) and a bottom connection (3).

6. Device for applying the method as claimed in one or more of the claims 1-3 and provided with an inoculation vessel as claimed in claims 4 and 5, which device contains furthermore a reaction chamber (37), which has a sealable connection (3,4,3') with the inoculation vessel,
**characterized by** a source (39) of sterile gas under a constant overpressure, which by means of a duct and valve system (49,48,56,57,58,59,60,5,3) is connectable to the inoculation vessel (1) and the reaction chamber (37), which source is provided with a gauge (42) in order to open the air exhaust valve (43) in case of a pressure higher than said constant overpressure.

7. Device as claimed in claim 6, **characterized in that** a sterile filter (41), that as a source of sterile gas is located in a duct of supplied air, is connected through a valve (49) with the duct and valve system and that the air exhaust valve (43) is located on that side of the filter (41) that is not facing the duct and valve system.

8. Device as claimed in claim 7, **characterized by** a feeder (45) for a disinfectant, which, through one or more valves (69,58), is connected to a duct (3) at the under side of the inoculation vessel (1), and a drain (66) for a disinfectant which, by means of valves (58,57), is connectable to the duct (3) at the under side of the inoculation vessel (1).

9. Device as claimed in claim 8, **characterized in that** one or more valves (57,58) connect to the source (39) of sterile gas with the duct (23) at the upper side of the inoculation vessel and valves (58,57) form the connection for the disinfectant from the duct (3) at the under side of the inoculation vessel (1) to the drain (66).

10. Device as claimed in one or more of the claims 6-9, provided with a feeder (38) and a drain (62) for a disinfectant, **characterized in that** a there is a device, reacting on a liquid level (48), which closes the feeder (46) of sterile gas, when the cleansing agent reaches a predetermined level on said device reacting on a liquid level (48), while there are additional valves or stop-valves (57,58,4,61) that direct the cleansing agent through the inoculation vessel and the reaction chamber and from there ensure its removal.

## Patentansprüche

1. Verfahren zur Inokulation einer Flüssigkeit, wobei das Inokulum placiert in einem oder mehreren geschlossenen Behältern (28) einem Inokulationsgefäss (1) hineingebracht wird, die Behälter werden im Inokulationsgefäss geöffnet und das Inokulum wird vom Inokulationsgefäss zu der Flüssigkeit in einem Reaktionsraum (37) transportiert, **dadurch gekennzeichnet**, dass das Inokulationsgefäss einem verschliessbaren Inokulationsgefäss (1) bildet, welches, nachdem die Behälter hineingebracht worden sind, geschlossen wird und, nach Desinfektion, mit sterilem Gas gefüllt wird, worauf das Inokulum dem Reaktionsraum hineingeführt wird.

2. Verfahren nach Anspruch 1, **dadurch gekennzeichnet**, dass das sterile Gas unter Druck sowohl dem Reaktionsraum (37) wie dem Inokulationsgefäss zugefügt wird.

3. Verfahren nach Anspruch 1 oder 2, betreffs der Desinfektion des Inokulationsgefässes, **dadurch gekennzeichnet**, dass die Desinfektion des Inokulationsgefässes von unten herab stattfindet via eine Leitung (3), welche das Inokulationsgefäss mit dem Reaktionsraum verbindet, wobei Zu- und Abfuhr (5) von einem Desinfektionsmittel stattfindet unmittelbar oben einen Verschluss (4) angeordnet in genannter Leitung.

4. Inokulationsgefäss zur Anwendung des Verfahrens nach einem oder mehreren der Ansprüche 1-3, versehen mit einer oder mehreren Festhaltvorrichtungen (27,29) für die Behälter (28) und einem oder mehreren Oeffnungsvorrichtungen, welche bewegbar sind hinsichtlich der Festhaltvorrichtungen, **dadurch gekennzeichnet**, dass das Inokulationsgefäss (1) eine oder mehrere hermetisch abschliessbare Oeffnungen (6) aufweist für das Hineinbringen von vollen und das Entfernen von leeren Behältern (28) und ein Mechanismus (25) um die Festhaltvorrichtungen und die Oeffnungsvorrichtungen hinsichtlich einander zu bewegen hindurch eine hermetisch abschliessbare Wand (24').

5. Inokulationsgefäss nach Anspruch 4, **dadurch gekennzeichnet,** dass das Vessel eine obere Verbindung (23) und eine untere Verbinding (3) aufweist.

6. Vorrichtung zur Anwendung des Verfahrens nach einem oder mehreren der Ansprüchen 1-3 und versehen mit einem Inokulationsgefäss nach Ansprüchen 4 und 5, welche Vorrichtung weiter einen Reaktionsraum (37) enthält, welcher eine abschliessbare Verbindung (3,4,3') mit dem Inokulationsgefäss hat, **gekennzeichnet durch** eine Quelle (39) von sterilem Gas unter einen konstanten Ueberdruck, welcher mittels eines Leitungs- und Ventilsystems (49,48,56,57,58,59,60,5,3) mit dem Inokulationsgefäss (1) und dem Reaktionsraum (37) verbindbar ist, welche Quelle mit einem Aufnehmer (42) versehen ist um die Luftabfuhr (43) zu Oeffnen im Falle eines höheren Drucks als den genannten konstanten Ueberdruck.

7. Vorrichtung nach Anspruch 6, **dadurch gekennzeichnet,** dass ein Sterilfilter (41), welcher wie eine Quelle von sterilen Gas in einer Leitung von zugeführter Luft liegt, mittels eines Ventils (49) verbunden ist mit dem Leitungsund Ventilsystem und dass das Luftabfuhrventil (43) sich an die Seite des Filters (41) befindet welche vom Leitungsund Ventilsystem abgekehrt ist.

8. Vorrichtung nach Anspruch 7, **gekennzeichnet durch** eine Zufuhr (45) für ein Desinfektionsmittel, welches mittels eines oder mehreren Ventile (69,58) mit der unteren Seite des Inokulationsgefäss (1) verbunden ist und eine Abfuhr (66) für ein Desinfektionsmittel, welches mittels Ventile (58,57) mit der Leitung (3) an die untere Seite des Inokulationsgefässes (1) verbindbar ist.

9. Vorrichtung nach Anspruch 8, **dadurch gekennzeichnet,** dass ein oder mehrere Ventile (57,58) eingerichtet sind die Quelle (39) des sterilen Gases zu verbinden mit der Leitung (23) an der obere Seite des Inokulationsgefässes und die Ventile (58,57) die Verbindung bilden für das Desinfektionsmittel von der Leitung (3) an der unteren Seite des Inokulationsgefässes (1) zur Abfuhr (66).

10. Vorrichtung nach einem oder mehreren der Ansprüchen 6-9 versehen mit einer Zufuhr (38) und einer Abfuhr (62) für eine Desinfektionsmittel, **dadurch gekennzeichnet**, dass eine Vorrichtung anwesend ist reagierend auf ein Flüssigkeitsniveau, welche die Zufuhr (46) des sterilen Gases abschliesst, wann die Reinigungsflüssigkeit eine vorabbestimmtes Niveau bei der genannten auf eine Flüssigkeitsniveau reagierenden Vorrichtung (48) erreicht, wobei weitere Ventile oder Verschlüsse (57,58,4,61) anwesend sind, die die Reinigungsflüssigkeit durch den Inokulationsgefäss und das Reaktionsraum leiten und daher deren Abfuhr gewährleisten.

## Revendications

1. Procédé d'inoculation d'un liquide, près de quoi l'inoculant placé dans un ou plusieurs supports fermés (28) est introduit dans un récipient d'inoculation (1), les supports sont ouverts à l'intérieur du récipient d'inoculation et l'inoculant du récipient d'inoculation est distribué au liquide présent dans une chambre de réaction (37) **caractérisé en ce** que le récipient d'inoculation forme une récipient d'inoculation (1) ferméable, quel récipient est fermé après les supports sont placés à l'intérieur et est rempli de gaz stérile après désinfection, après quoi l'inoculant est alimenté à la chambre de réaction.

2. Procédé suivant la revendication 1, **caractérisé en ce** que le gaz stérile est alimenté sous pression à la chambre de réaction (37) et aussi au récipient d'inoculation (1).

3. Procédé suivant la revendication 1 ou 2, concernant la désinfection du récipient d'inoculation, **caractérisé en ce** que la désinfection du récipient d'inoculation se passe d'en bas par un conduit (3) quel connecte le récipient d'inoculation avec la chambre de réaction, pendant que l'arrivée et la sortie (5) d'inoculant se passe immédiatement au-dessus d'une soupape (4) dans ledit conduit.

4. Récipient d'inoculation pour application le procédé suivant une ou plusieurs des revendications 1-3, prévu d'un ou plusieurs des dispositifs de maintien (27,29) pour les conteneurs (28) et un ou plusieurs des dispositifs d'ouvertures mobiles à l'égard des dispositifs de maintain **caractérisé en ce** que le récipient d'inoculation (1) présente une ou plusieurs d'ouvertures hermétiquement ferméables (6) pour introduire des conteneurs remplis et pour éloigner des conteneurs vides (28) et un mécanisme (25) de mettre en mouvement les dispositifs de maintien et les dispositifs d'ouvertures à l'égard de l'un à l'autre à travers d'un paroi hermétiquement ferméable (24').

5. Récipient d'inoculation suivant la revendication 4, **caractérisé en ce** qu'il compris une connexion supérieure (23) et une connexion inférieure (3).

6. Dispositif pour l'application le procédé suivant une ou plusieurs des revendications 1-3 et prévu d'un récipient d'inoculation suivant les reveidications 4 et 5, quel dispositif contient de plus une chambre de réaction (37) ayant une connexion ferméable (3,4,3') avec le récipient d'inoçulation, **caractérisé par** une source de gaz stérile sous surpression constante, quelle est connectable au moyen d'un système des conduits et des soupapes (49,48,56,57,58,59,60,5,3) avec le récipient d'inoculation (1) et la chambre de réaction (37), quelle source est prévue d'un capteur (42) afin d'ouvrir la soupape d'échappement d'air (43) en cas d'une pression plus haut que ladite surpression constante.

7. Dispositif suivant la revendication 6, **caractérisé en ce** qu'une filtre stérile (41), placée dans un conduit d'air ammené comme une source du gaz stérile, est connectée par une soupape (49) avec le système des conduits et des soupapes et que la soupape d'échappement d'air (43) se trouve à ce côté du filtre (41) détournant du système des conduits et des soupapes.

8. Dispositif suivant la revendication 7, **caractérisé par** une distribution (45) pour un désinfectant, quel, par une ou plusiers des soupapes (69,58), est connectée à un conduit (3) au côté inférieur du récipient d'inoculation (1) et un conduit de sortie (66) pour le désinfectant, quel, par moyen des soupapes (58,57) est connectable au conduit (3) au côté inférieur du récipient d'inoculation (1).

9. Dispositif suivant la revendication 8, **caractérisé en ce** qu'une ou plusieurs des soupapes (57,58) sont arrangés de connecter la source (39) du gaz stérile avec le conduit (23) au côté supérieur du récipient d'inoculation et les soupapes (58,57) se forment la connexion pour le désinfectant à partir du conduit (3) au côté inférieur du récipient d'inoculation (1) vers le conduit de sortie (66).

10. Dispositif suivant l'une ou plusieurs des revendications 6-9, prévu d'une arrivée (38) et une sortie (62) pour un désinfectant, **caractérisé en ce** qu'il y a un dispositif réagant sur un niveau de liquide (48), quel ferme l'alimentation (46) du gaz stérile, quand l'agent de nettoyage atteint un niveau prédeterminé sur ce dispositif réagant sur un niveau de liquide (48), pendant que des soupapes ou des soupapes d'arrêt additives (57,58,4,61) sont présents, quels guident l'agent de nettoyage à travers le récipient d'inoculation et la chambre de réaction et assurent leur renvoi de là.
